# EUROPÄISCHE PATENTSCHRIFT

(11) **EP 0 977 545 B1**
(45) Veröffentlichungstag und Bekanntmachung des Hinweises auf die Patenterteilung: **16.02.2005**
(21) Anmeldenummer: 98920524.0
(22) Anmeldetag: 15.04.1998
(51) Int. Cl.: A61K 7/13

(54) **VERWENDUNG VON 1-SUBSTITUIERTEN ISATINEN ZUM FÄRBEN VON KERATINHALTIGEN FASERN**
THE USE OF 1-SUBSTITUTED ISATINS TO DYE FIBERS CONTAINING KERATIN
UTILISATION D'ISATINES 1-SUBSTITUEES POUR LA COLORATION DE FIBRES KERATINIQUES

(30) Priorität: 24.04.1997 DE 19717282
(43) Veröffentlichungstag der Anmeldung: 09.02.2000
(73) Patentinhaber: Henkel Kommanditgesellschaft auf Aktien, 40589 Düsseldorf-Holthausen (DE)
(72) Erfinder: MÖLLER, Hinrich, D-40789 Monheim (DE); ROSE, David, D-40723 Hilden (DE); HÖFFKES, Horst, D-40595 Düsseldorf (DE); MEINIGKE, Bernd, D-51381 Leverkusen (DE)
(86) Internationale Anmeldenummer: PCT/EP1998/002199
(87) Internationale Veröffentlichungsnummer: WO 1998/047472

(56) Entgegenhaltungen:
- WO-A-93/19725
- WO-A-94/24988
- WO-A-95/24886

## Beschreibung

Die Erfindung betrifft die Verwendung von 1-substituierten Isatinen zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkompanenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxyethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2'-hydroxyethyl)-pyrazol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert; ihr Nachteil liegt jedoch darin, daß die Färbungen häufig nur über unzureichende Echtheitseigenschaften verfügen.

In den internationalen Patentanmeldungen WO 93/19725, WO 94/24988, WO 94/24989 und WO 95/24886 werden Mittel zum Färben von keratinhaltigen Fasern offenbart, die als Farbstoffkomponente Isatinderivate enthalten.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z.B. H₂O₂ angewiesen zu sein. Darüber hinaus sollen die Färbemittel kein oder lediglich ein sehr geringes Sensibilisierungspotential aufweisen.

Überraschenderweise wurde nun gefunden, daß sich bestimmte Isatin-Derivate auch in Abwesenheit von oxidierenden Agentien hervorragend zum Färben von keratinhaltigen Fasern eignen. Sie ergeben Ausfärbungen mit hervorragender Brillianz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist die Verwendung von 1-substituierten Isatinen mit der Formel I

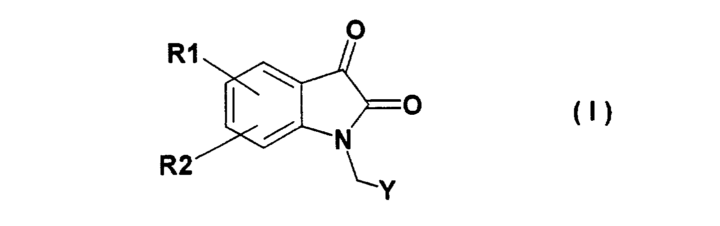

in der R¹ und R² unabhängig voneinander ein Wasserstoffatom, ein Halogenatom, eine Hydroxy-, eine (C₁-C₄)-Alkyl-, Hydroxy(C₁-C₄)-alkyl-, tert.-Amino(C₁-C₄)-alkyl-, (C₁-C₄)-Alkoxy-, eine Aminogruppe, die durch ein oder zwei (C₁-C₄)-Alkyl- oder Hydroxy-(C₁-C₄)-alkylgruppen substituiert sein kann, eine Nitro-, Carboxy- oder eine Sulfogruppe bedeuten, und
Y eine Hydroxygruppe, eine (C₁-C₄)-Alkoxygruppe oder eine Aminogruppe bedeutet, die durch (C₁-C₄)-Alkyl-, (C₁-C₄)-Alkoxy-(C₁₋C₄)-alkyl-, Carboxy-(C₁-C₄)-alkyl-, Sulfo-(C₁-C₄)-alkyl- oder Hydroxy-(C₁-C₄)-alkylgruppen substituiert oder Bestandteil eines heterocyclischen 5-, 6-, oder 7-Ringes sein kann,
oder physiologisch verträglichen Salzen davon zum Färben von keratinhaltigen Fasern, insbesondere menschlichen Haaren.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Die Isatin-Derivate der Formel I sind vorzugsweise ausgewählt aus der Gruppe 1-Hydroxymethylisatin, 1-Hydroxymethyl-5-methylisatin, 1-Hydroxymethyl-5-chlorisatin, 1-Hydroxymethyl-5-sulfoisatin, 1-Hydroxymethyl-5-carboxyisatin, 1-Hydroxymethyl-5-nitroisatin, 1-Hydroxymethyl-5-bromisatin, 1-Hydroxymethyl-5-methoxyisatin, 1-Hydroxymethyl-5,7-dichlorisatin, 1-Dimethylaminomethylisatin, 1-Diethylaminomethylisatin, 1-(Bis-(2-hydroxyethyl)-aminomethyl)-isatin, 1-(2-Hydroxyethylaminomethyl)-isatin, 1-(Bis-(2-hydroxypropyl)-aminomethyl)-isatin, 1-Pyrrolidinomethylisatin, 1-Piperidinomethylisatin, 1-Morpholinomethylisatin, 1-(1,2,4-Triazolyl)-methylisatin, 1-(1-Imidazolyl)-methylisatin, 1-Carboxymethylaminomethylisatin, 1-(2-Carboxyethylaminomethyl)-isatin, 1-(3-Carboxypropylaminomethyl)-isatin, 1-(Bis-(2-hydroxyethyl)-aminomethyl)-5-methylisatin, 1-Piperidinomethyl-5-chlorisatin, 1-(2-Sulfoethylamino)-isatin, sowie die Alkali- und gegebenenfalls Ammoniumsalze der sauren Verbindungen, wobei 1-Hydroxymethylisatin, 1-Hydroxymethyl-5-methylisatin, 1-Hydroxymethyl-5-chlorisatin, 1-Diethylaminomethylisatin, 1-(Bis-(2-hydroxyethyl)-aminomethyl)-isatin, 1-Pyrrolidinomethylisatin, 1-Piperidinomethylisatin, 1-Morpholinomethylisatin und 1-(3-Carboxypropylaminomethyl)-isatin besonders bevorzugt sind.

Die Verbindungen mit der Formel I sind literaturbekannt oder im Handel erhältlich.

Färbemittel, die als färbende Komponente Isatinderivate der Formel I allein enthalten, werden bevorzugt für Färbungen im Gelbbereich eingesetzt. Färbungen mit noch erhöhter Brillanz und weiter verbesserten Echtheitseigenschaften, vor allem im Orange-, Braun-, Violett- und Schwarzbereich, werden erzielt, wenn die Isatin-Derivate der Formel I gemeinsam mit Verbindungen mit primärer oder sekundärer Aminogruppe, z.B. Anilinderivaten, mit Stickstoff enthaltenden heterocyclischen Verbindungen, z.B. primären heteroaromatischen Aminen, aromatischen Hydroxyverbindungen oder CH-aktiven Verbindungen, verwendet werden. Dies sind einerseits Verbindungen, die für sich alleine keratinhaltige Fasern nur schwach färben und erst gemeinsam mit den Isatinen der Formel I brillante Färbungen ergeben. Darunter sind darunter aber auch Verbindungen, deren Einsatz als Oxidationsfarbstoffvorprodukte bekannt ist.

Die Isatin-Derivate der Formel I werden vorzugsweise in einer Menge von jeweils 0,03 bis 65 mmol, insbesondere 1 bis 40 mmol, jeweils bezogen auf 100 g des gesamten Färbemittels, verwendet. Sie können als direktziehende Färbemittel oder in Gegenwart von Oxidationsfarbstoffvorprodukten eingesetzt werden.

Ein weiterer Gegenstand der vorliegenden Erfindung ist ein Mittel zum Färben von keratinhaltigen Fasern, insbesondere von menschlichen Haaren, das
A mindestens ein Isatin-Derivat mit der obigen Formel I und
B mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
enthält.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Isatin-Derivate der Formel I gemeinsam zum Einsatz komen; ebenso können auch mehrere verschiedene Verbindungen der Komponente B gemeinsam verwendet werden. Unter diese Ausführungsform fällt auch die Verwendung von solchen Substanzen, die Reaktionsprodukte von Isatinderivaten der Formel I mit den genannten Verbindungen B darstellen.

Geeignete Verbindungen mit primärer oder sekundärer Aminogruppe sind z.B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, m-, p-Phenylendiamin, o-, m-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 5-(2-Hydroxyethylamino)-4-methoxy-2-methyl-phenol, 1,3-Diamino-2,4-dimethoxybenzol, 2-, 3-, 4-Aminobenzoesäure, - phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest , wie sie in der Formel II dargestellt sind, 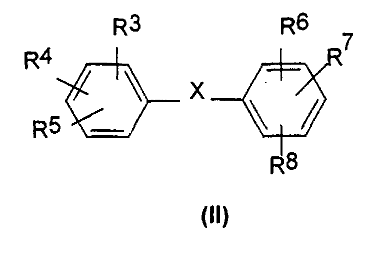 in der R³ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht, R⁴, R⁵, R⁶, R⁷ und R⁸ für Wasserstoff, eine Hydroxy- oder eine Aminogruppe, die durch C₁₋₄-Alkyl-, C₁₋₄-Hydroxyalkyl-, C₁₋₄-Alkoxy-, C₁₋₄-Aminoalkyl- oder C₁₋₄-Alkoxy-C₁₋₄-alkylgruppen substituiert sein kann, oder für eine Sulfonsäuregruppe stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

**Z-(CH**_{**2**}**-Q-CH**_{**2**}**-Z')**_{**o**} (III)

in der Q eine direkte Bindung, eine CH₂- oder CHOH-Gruppe bedeutet,
Z und Z' unabhängig voneinander für ein Sauerstoffatom, eine NR⁹-Gruppe, worin R⁹ Wasserstoff, eine C₁₋₄-Alkyl- oder eine Hydroxy-C₁₋₄-alkylgruppe bedeutet,
die Gruppe O-(CH₂)ₚ-NH oder NH-(CH₂)_{p'} O- worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-monooder -di-Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Satz- oder Schwefelsäure, eingesetzt werden.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Geeignete aromatische Hydroxyverbindungen sind z.B. 2-, 4-, 5-Methylresorcin, Resorcin, 2,5-Dimethylresorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethylindolium-p-toluolsulfonat, 1,2,3,3-Tetramethyl-3H-indolium-methansulfonat, 2,3-Dimethyl-benzothiazoliumiodid, 2,3-Dimethyl-benzothiazolium-p-toluolsulfonat, Rhodanin, Rhodanin-3-essigsäure, 1-Ethyl-2-chinaldiniumiodid, 1-Methyl-2-chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Die Komponente B wird besonders bevorzugt ausgewählt aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chior-, 2-Aminomethyl-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salze.

Auf die Anwesenheit von Oxidationsmitteln, z.B. H₂O₂, kann dabei verzichtet werden. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer weiteren bevorzugten Ausführungsform enthalten die erfindungsgemäßen Färbemittel zur weiteren Modifizierung der Farbnuancen neben den erfindungsgemäß enthaltenen Verbindungen und ggf. weiteren Oxidationsfarbstoffvorprodukten zusätzlich übliche direktziehende Farbstoffe, z.B. aus der Gruppe der Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Es ist nicht erforderlich, daß die Verbindungen der Komponete B und ggf. weitere Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine - COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Laurylcümethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethyisilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Zum Färben der keratinhaltigen Fasern, insbesondere zum Färben von menschlichen Haaren, werden die Färbemittel in der Regel in Form des wasserhaltigen, kosmetischen Trägers in einer Menge von 100 g auf das Haar aufgebracht, ca. 30 Minuten dort belassen und anschließend ausgespült oder mit einem handelsüblichen Haarshampoo ausgewaschen.

Die Isatin-Derivate der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Isatine der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Herstellung einer Färbelösung

Es wurde eine Aufschlämmung von 10 mMol eines Isatinderivats der Formel I, 10 mMol einer Aminoverbindung, 10 mmol Natriumacetat und einen Tropfen einer 20 %igen Fettalkylethersulfat-Lösung in 100 ml Wasser bereitet. Die Aufschlämmung wurde kurz auf ca. 80°C erhitzt und nach dem Abkühlen filtriert, der pH-Wert wurde anschließend auf 6 eingestellt.

In diese Färbelösung wurde bei 30°C 30 Minuten lang eine Strähne zu 90% ergrauten, nicht vorbehandelten Menschenhaares eingebracht. Die gefärbte Strähne wurde dann 30 Sek. mit lauwarmem Wasser gespült, im warmen (30-40°C) Luftstrom getrocknet und anschließend ausgekämmt. Danach wurden die Ausfärbungen visuell bei Tageslicht beurteilt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **Ausfärbungen mit 1-Pyrrolidinomethylisatin** | | |
|---|---|---|
| Komponente B | Färbenuance | |
| | Farbtiefe | |
| - | gelb | ++ |
| 2,5-Diaminotoluol x H₂SO₄ | violettrot | |
| | | ++(+) |
| 2,4,5,6-Tetraaminopyrimidin x H₂SO₄ | rot | |
| | | +++ |
| 1,8-Bis-(2,5-diaminophenoxy)-3,6-dioxaoctan x 4 HCl | rotviolett | |
| | | +++ |
| 2-(2,5-Diaminophenyl)-ethanol x H₂SO₄ | violettrot | |
| | | +++ |
| 2-Aminomethyl-4-aminophenol x 2HCl | braunorange | ++ |
| N, N-Bis-(2-hydroxyethyl)-p-phenylendiamin x H₂SO₄ | dunkelviolett | |
| | | +++ |
| 4,4'-Diaminodiphenylamin x H₂SO₄ | dunkelolivgrün | ++ |

**Tabelle 2**

| **Ausfärbungen mit 1-Morpholinomethylisatin** | | |
|---|---|---|
| Komponente B | Färbenuance | |
| | Farbtiefe | |
| 2,5-Diaminotoluol x H₂SO₄ | violettrot | |
| | | ++(+) |
| 2,4,5,6-Tetraaminopyrimidin x H₂SO₄ | rot | ++ |
| 2-Methylamino-3-amino-6-methoxypyridin x 2HCl | gelborange | ++ |
| 2-(2,5-Diaminophenyl)-ethanol x H₂SO₄ | violettrot | ++ |
| N,N-Bis-(2-hydroxyethyl)-p-phenylendimin x H₂SO₄ | dunkelviolett | |
| | | +++ |
| 4,4'-Diaminodiphenylamin x H₂SO₄ | blauschwarz | |
| | | +++ |
| 2,6-Dimethoxy-3,5-diaminopyridin x 2HCl | dunkelgrau | |
| | | ++(+) |

**Tabelle 3**

| **Ausfärbungen mit 1-(3-Carboxypropylaminomethyl)-isatin** | | |
|---|---|---|
| Komponente B | Färbenuance | |
| | Farbtiefe | |
| - | gelb | ++ |
| 2,5-Diaminotoluol x H₂SO₄ | violettrot | |
| | | ++(+) |
| 2,4,5,6-Tetraaminopyrimidin x H₂SO₄ | kupfer | |
| | | ++(+) |
| 2-Methylamino-3-amino-6-methoxypyridin x 2HCl | grauviolett | ++ |
| 2-(2,5-Diaminophenyl)-ethanol x H₂SO₄ | rotviolett | |
| | | ++(+) |
| 2-Aminomethyl-4-aminophenol x 2HCl | braunorange | |
| | | ++(+) |
| N,N-Bis-(2-hydroxyethyl)-p-phenylendiamin x H₂SO₄ | schwarzviolett | |
| | | +++ |
| 4,4'-Diaminodiphenylamin x H₂SO₄ | dunkelviolett | |
| | | ++(+) |
| 2,6-Dimethoxy-3,5-diaminopyridin x 2HCl | schwarz | |
| | | +++ |

**Tabelle 4**

| **Ausfärbungen mit 1-Piperidinomethylisatin** | | |
|---|---|---|
| Komponente B | Färbenuance | |
| | Farbtiefe | |
| - | gelb | |
| | | ++(+) |
| 2,5-Diaminotoluol x H₂SO₄ | rotviolett | |
| | | ++(+) |
| 2,4,5,6-Tetraaminopyrimidin x H₂SO₄ | violettrot | |
| | | ++(+) |
| 1,8-Bis-(2,5-diaminophenoxy)-3,6-dioxaoctan x 4 HCl | violettrot | |
| | | ++(+) |
| 2-(2,5-Diaminophenyl)-ethanol x H₂SO₄ | violettrot | |
| | | ++(+) |
| 2-Aminomethyl-4-aminophenol x 2HCl | orangebraun | ++ |
| N,N-Bis-(2-hydroxyethyl)-p-phenylendiamin x H₂SO₄ | dunkelviolett | |
| | | +++ |
| 4,4'-Diaminodiphenylamin x H₂SO₄ | schwarz | |
| | | +++ |
| 2,6-Dimethoxy-3,5-diaminopyridin x 2HCl | dunkelgrau | |
| | | ++(+) |

**Tabelle 5**

| **Ausfärbungen mit 1-Diethylaminomethylisatin** | | |
|---|---|---|
| Komponente B | Färbenuance | |
| | Farbtiefe | |
| - | reingelb | |
| | | ++(+) |
| 2,5-Diaminotoluol x H₂SO₄ | kupfer | ++ |
| 2,4,5,6-Tetraaminopyrimidin x H₂SO₄ | rot | ++ |
| 2-(2,5-Diaminophenyl)-ethanol x H₂SO₄ | rotbraun | ++ |
| 2-Aminomethyl-4-aminophenol x 2HCl | orangebraun | ++ |
| N, N-Bis-(2-hydroxyethyl)-p-phenylendiamin x H₂SO₄ | dunkelrotviolett | |
| | | +++ |
| 2,6-Dimethoxy-3,5-diaminopyridin x 2HCl | dunkelolivgrün | ++ |

**Tabelle 6**

| **Ausfärbungen mit 1-Bis-(2-hydroxyethyl)-aminomethylisatin** | | |
|---|---|---|
| Komponente B | Färbenuance | |
| | Farbtiefe | |
| - | goldgelb | |
| | | ++(+) |
| 2,5-Diaminotoluol x H₂SO₄ | rotviolett | |
| | | +++ |
| 2,4,5,6-Tetraaminopyrimidin x H₂SO₄ | rot | |
| | | ++(+) |
| 1,8-Bis-(2,5-diaminophenoxy)-3,6-dioxaoctan x 4 HCl | braunviolett | |
| | | +++ |
| 2-(2,5-Diaminophenyl)-ethanol x H₂SO₄ | violettrot | |
| | | ++(+) |
| 2-Aminomethyl-4-aminophenol x 2HCl | braunorange | ++ |
| N,N-Bis-(2-hydroxyethyl)-p-phenylendiamin x H₂SO₄ | dunkelviolett | |
| | | +++ |
| 4,4'-Diaminodiphenylamin x H₂SO₄ | dunkelviolett | |
| | | +++ |
| 2,6-Dimethoxy-3,5-diaminopyridin x 2HCl | dunkelbraun | |
| | | +++ |

**Tabelle 7**

| **Ausfärbungen mit 1-Hydroxymethylisatin** | | |
|---|---|---|
| Komponente B | Färbenuance | |
| | Farbtiefe | |
| - | goldgelb | ++ |
| 2,5-Diaminotoluol x H₂SO₄ | violettrot | |
| | | ++(+) |
| 2,4,5,6-Tetraaminopyrimidin x H₂SO₄ | rostrot | |
| | | ++(+) |
| 2-Methylamino-3-amino-6-methoxypyridin x 2HCl | violett | |
| | | ++(+) |
| 2-(2,5-Diaminophenyl)-ethanol x H₂SO₄ | braunviolett | |
| | | ++(+) |
| 2-Aminomethyl-4-aminophenol x 2HCl | gelbbraun | ++ |
| N, N-Bis-(2-hydroxyethyl)-p-phenylendiamin x H₂SO₄ | dunkelviolett | |
| | | +++ |
| 2,6-Dimethoxy-3,5-diaminopyridin x 2HCl | dunkelbraun | |
| | | ++(+) |

## Patentansprüche

1. Verwendung von Isatin-Derivaten mit der Formel I 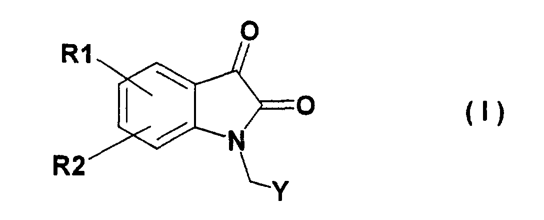 in der R¹ und R² unabhängig voneinander ein Wasserstoffatom, ein Halogenatom, eine Hydroxy-, eine (C₁-C₄)-Alkyl-, Hydroxy(C₁-C₄)-alkyl-, tert.-Amino(C₁-C₄)-alkyl-, (C₁-C₄)-Alkoxy-, eine Aminogruppe, die durch ein oder zwei (C₁-C₄)-Alkyl- oder Hydroxy-(C₁-C₄)-alkylgruppen substituiert sein kann, eine Nitro-, Carboxy- oder eine Sulfogruppe bedeuten, und
Y eine Hydroxygruppe, eine (C₁-C₄)-Alkoxygruppe oder eine Aminogruppe bedeutet, die durch (C₁-C₄)-Alkyl-, (C₁-C₄)-Alkoxy-(C₁₋C₄)-alkyl-, Carboxy-(C₁-C₄)-alkyl-, Sulfo-(C₁-C₄)-alkyl- oder Hydroxy-(C₁-C₄)-alkylgruppen substituiert oder Bestandteil eines heterocyclischen 5-, 6- oder 7-Ringes sein kann,
oder physiologisch verträglichen Salzen davon zum Färben von keratinhaltigen Fasern, insbesondere menschlichen Haaren.

2. Verwendung nach Anspruch 1, **dadurch gekennzeichnet, daß** die Isatin-Derivate der Formel I in einer Menge von jeweils 0,03 bis 65, insbesondere 1 bis 40 mmol, jeweils bezogen auf 100 g des gesamten Färbemittels, enthalten sind.

3. Verwendung nach Anspruch 1 oder 2, **dadurch gekennzeichnet, daß** das Isatin-Derivat der Formel I ausgewählt ist aus 1-Hydroxymethylisatin, 1-Hydroxymethyl-5-methylisatin, 1-Hydroxymethyl-5-chlorisatin, 1-Hydroxymethyl-5-sulfoisatin, 1-Hydroxymethyl-5-carboxyisatin, 1-Hydroxymethyl-5-nitroisatin, 1-Hydroxymethyl-5-bromisatin, 1-Hydroxymethyl-5-methoxyisatin, 1-Hydroxymethyl-5,7-dichlorisatin, 1-Dimethylaminomethylisatin, 1-Diethylaminomethylisatin, 1-(Bis-(2-hydroxyethyl)-aminomethyl)-isatin, 1-(2-Hydroxyethylaminomethyl)-isatin, 1-(Bis-(2-hydroxypropyl)-aminomethyl)-isatin, 1-Pyrrolidinomethylisatin, 1-Piperidinomethylisatin, 1-Morpholinomethylisatin, 1-(1,2,4-Triazolyl)-methylisatin, 1-(1-Imidazolyl)-methylisatin, 1-Carboxymethylaminomethylisatin, 1-(2-Carboxyethylaminomethyl)-isatin, 1-(3-Carboxypropylaminomethyl)-isatin, 1-(Bis-(2-hydroxyethyl)-aminomethyl)-5-methylisatin, 1-Piperidinomethyl-5-chlorisatin, 1-(2-Sulfoethylamino)-isatin, sowie die Alkali- und Ammoniumsalze der sauren Verbindungen.

4. Verwendung nach einem der Ansprüche 1 bis 3, **dadurch gekennzeichnet, daß** zusätzlich mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung eingesetzt wird.

5. Verwendung nach einem der Ansprüche 1 bis 4, **dadurch gekennzeichnet, daß** Oxidationsmittel in einer Menge von 0,01 bis 6 Gew.-%, bezogen auf die Anwendungslösung, eingesetzt werden.

6. Verwendung nach Anspruch 5, **dadurch gekennzeichnet, daß** als Oxidationsmittel H₂O₂ eingesetzt wird.

7. Verwendung nach einem der Ansprüche 1 bis 6, **dadurch gekennzeichnet, daß** anionische, zwitterionische und/oder nichtionische Tenside eingesetzt werden.

8. Mittel zum Färben von keratinhaltigen Fasern, insbesondere menschlichen Haaren, enthaltend
A mindestens ein Isatin-Derivat mit der Formel I, 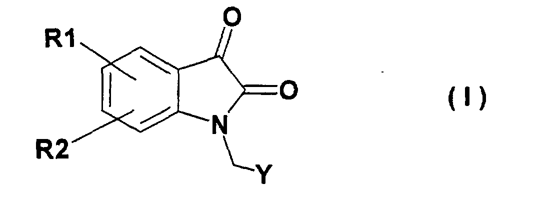 in der R¹ und R² unabhängig voneinander ein Wasserstoffatom, ein Halogenatom, eine Hydroxy-, eine (C₁-C₄)-Alkyl-, Hydroxy(C₁-C₄)-alkyl-, tert.-Amino(C₁-C₄)-alkyl-, (C₁-C₄)-Alkoxy-, eine Aminogruppe, die durch ein oder zwei (C₁-C₄)-Alkyl- oder Hydroxy-(C₁-C₄)-alkylgruppen substituiert sein kann, eine Nitro-, Carboxy- oder eine Sulfogruppe bedeutet, und
Y eine Hydroxygruppe, eine (C₁-C₄)-Alkoxygruppe oder eine Aminogruppe bedeutet, die durch (C₁-C₄)-Alkyl-, (C₁-C₄)-Alkoxy-(C₁₋C₄)-alkyl-, Carboxy-(C₁-C₄)-alkyl-, Sulfo-(C₁-C₄)-alkyl- oder Hydroxy-(C₁-C₄)-alkylgruppen substituiert oder Bestandteil eines heterocyclischen 5-, 6- oder 7-Ringes sein kann,
oder physiologich verträgliche Salze davon und
B mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung.

9. Mittel nach Anspruch 8, **dadurch gekennzeichnet, daß** die Komponente B ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 4-Amino-2-hydroxymethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfansäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, - sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propantetrahydrochlorid, 1,8-Bis-(2,5-diaminophenoxy)-3,6-dioxaoctan-tetrahydrochlorid, 1,3-Bis-(4-aminophenytamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und
CH-aktiven Verbindungen ausgewählt aus der Gruppe, bestehend aus 1,2,3,3-Tetramethyl-3H-indoliniumiodid, 1,2,3,5-Tetramethylindolinium-p-toluolsulfonat, 1,2,3,5-Tetramethylindolinium-methansulfonat, 2,3-Dimethyl-benzothiazoliumiodid, 2,3-Dimethyl-benzothiazolium-p-toluolsulfonat, Rhodanin, Rhodanin-3-essigsäure, 1-Ethyl(Methyl)-2-chinaldiniumiodid, Barbitursäure, Thiobarbitursäure, 1,3-Dimethyl(ethyl)thiobarbitursäure, Oxindol, Cumaranon und 1-Methyl-3-phenyl-2-pyrazolinon.

10. Mittel nach Anspruch 9, **dadurch gekennzeichnet, daß** die Komponente B ausgewählt ist aus der Gruppe bestehend aus N-(2-Hydroxyethyl)-N-ethyl-, 2-Chlor-p-phenylendiamin, N,N-Bis-(2-hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

11. Mittel nach eienm der Ansprüche 8 bis 10, **dadurch gekennzeichnet, daß** direktziehende Farbstoffe aus der Gruppe der Nitrophenylendiamine, Nitroaminophenole, Anthrachinone oder Indophenole eingesetzt werden.

12. Mittel nach einem der Ansprüche 8 bis 11, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

13. Verfahren zum Färben von keratinhaltigen Fasern, worin ein Färbemittel, enthaltend
A mindestens ein Isatin-Derivat der Formel I 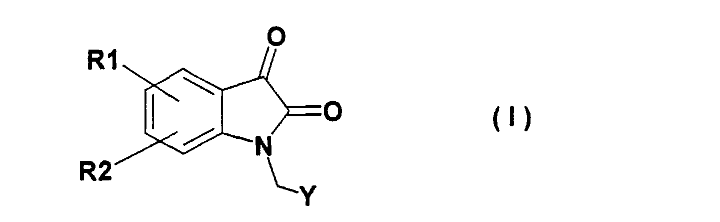 in der R¹ und R² unabhängig voneinander ein Wasserstoffatom, ein Halogenatom, eine Hydroxy-, eine (C₁-C₄)-Alkyl-, Hydroxy(C₁-C₄)-alkyl-, tert.-Amino(C₁-C₄)-alkyl-, (C₁-C₄)-Alkoxy-, eine Aminogruppe, die durch ein oder zwei (C₁-C₄)-Alkyl- oder Hydroxy-(C₁-C₄)-alkylgruppen substituiert sein kann, eine Nitro-, Carboxy- oder eine Sulfogruppe bedeutet, und
Y eine Hydroxygruppe, eine (C₁-C₄)-Alkoxygruppe oder eine Aminogruppe bedeutet, die durch (C₁-C₄)-Alkyl-, (C₁-C₄)-Alkoxy-(C₁₋C₄)-alkyl-, Carboxy-(C₁-C₄)-alkyl-, Sulfo-(C₁-C₄)-alkyl- oder Hydroxy-(C₁-C₄)-alkylgruppen substituiert oder Bestandteil eines heterocyclischen 5-, 6- oder 7-Ringes sein kann,
oder physiologich verträgliche Salze davon und
B mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden, aromatischen Hydroxyverbindungen, und/oder mindestens eine CH-aktive Verbindung,
sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. The use of isatin derivatives corresponding to formula (I): 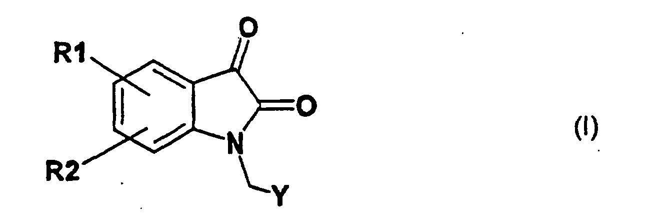 in which R¹ and R² independently of one another represent a hydrogen atom, a halogen atom, a hydroxy group, a (C₁₋₄)-alkyl, hydroxy-(C₁₋₄)-alkyl, tert.amino-(C₁₋₄)-alkyl, (C₁₋₄)-alkoxy group, an amino group optionally substituted by one or two (C₁₋₄)-alkyl or hydroxy-(C₁₋₄)-alkyl groups, a nitro, carboxy or sulfo group and
Y is a hydroxy group, a (C₁₋₄)-alkoxy group or an amino group which may be substituted by (C₁₋₄)-alkyl, (C₁₋₄)-alkoxy-(C₁₋₄)-alkyl, carboxy-(C₁₋₄)-alkyl, sulfo-(C₁₋₄)-alkyl or hydroxy-(C₁₋₄)-alkyl groups or which may be part of a heterocyclic 5-, 6- or 7-membered ring,
or physiologically compatible salts thereof for coloring keratin-containing fibers, more especially human hair.

2. The use claimed in claim 1, **characterized in that** the isatin derivatives corresponding to formula I are present in a quantity of 0.03 to 65 mmol and more especially in a quantity of 1 to 40 mmol, based on 100 g of the colorant as a whole.

3. The use claimed in claim 1 or 2, **characterized in that** the istain derivative corresponding to formula I is selected from the group consisting of 1-hydroxymethyl isatin, 1-hydroxymethyl-5-methyl isatin, 1-hydroxymethyl-5-chloroisatin, 1-hydroxymethyl-5-sulfoisatin, 1-hydroxymethyl-5-carboxyisatin, 1-hydroxymethyl-5-nitroisatin, 1-hydroxymethyl-5-bromoisatin, 1-hydroxymethyl-5-methoxyisatin, 1-hydroxymethyl-5,7-dichloroisatin, 1-dimethylaminomethyl isatin, 1-diethylaminomethyl isatin, 1-(bis-(2-hydroxyethyl)-aminomethyl)-isatin, 1-(2-hydroxyethylaminomethyl)-isatin, 1-(bis-(2-hydroxypropylyaminomethyl)-isatin, 1-pyrrolidinomethyl isatin, 1-piperidinomethyl isatin, 1-morpholinomethyl isatin, 1-(1,2,4-triazolyl)-methyl isatin, 1-(1-imidazolyl)-methyl isatin, 1-carboxymethylaminomethyl isatin, 1-(2-carboxyethylaminomethyl)-isatin, 1-(3-carboxypropylaminomethyl)-isatin, 1-(bis-(2-hydroxyethyl)-aminomethyl)-5-methyl isatin, 1-piperidinomethyl-5-chloroisatin, 1-(2-sulfoethylamino)-isatin and the alkali metal and optionally ammonium salts of the acidic compounds.

4. The use claimed in any of claims 1 to 3, **characterized in that** they additionally contain at least one compound containing a primary or secondary amino group or hydroxy group selected from primary or secondary aliphatic or aromatic amines, nitrogen-containing heterocyclic compounds, amino acids, oligopeptides made up of 2 to 9 amino acids and aromatic hydroxy compounds and/or at least one CH-active compound.

5. The use claimed in any of claims 1 to 4, **characterized in that** oxidizing agents are used in a quantity of 0.01 to 6% by weight, based on the solution applied.

6. The use claimed in claim 5, **characterized in that** H₂O₂ is used as the oxidizing agent.

7. The use claimed in any of claims 1 to 6, **characterized in that** anionic, zwitterionic and/or nonionic surfactants are used.

8. A preparation for coloring keratin-containing fibers, more especially human hair, containing
A at least one isatin derivative corresponding to general formula I: 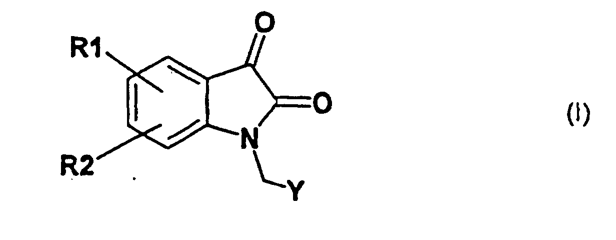 in which R¹ and R² independently of one another represent a hydrogen atom, a halogen atom, a hydroxy group, a (C₁₋₄)-alkyl, hydroxy-(C₁₋₄)-alkyl, tert.amino-(C₁₋₄)-alkyl, (C₁₋₄)-alkoxy group, an amino group optionally substituted by one or two (C₁₋₄)-alkyl or hydroxy-(C₁₋₄)-alkyl groups, a nitro, carboxy or sulfo group and
Y is a hydroxy group, a (C₁₋₄)-alkoxy group or an amino group which may be substituted by (C₁₋₄)-alkyl, (C₁₋₄)-alkoxy-(C₁₋₄)-alkyl, carboxy-(C₁₋₄)-alkyl, sulfo-(C₁₋₄)-alkyl or hydroxy-(C₁₋₄)-alkyl groups or which may be part of a heterocyclic 5-, 6- or 7-membered ring,
or physiologically compatible salts thereof and
B at least one compound containing a primary or secondary amino group or hydroxy group selected from primary or secondary aliphatic or aromatic amines, nitrogen-containing heterocyclic compounds, amino acids, oligopeptides made up of 2 to 9 amino acids and aromatic hydroxy compounds and/or at least one CH-active compound.

9. A preparation as claimed in claim 8, **characterized in that** component B is selected from primary or secondary amines from the group consisting of N-(2-hydroxyethyl)-N-ethyl-, N-(2-methoxyethyl)-, 2,3-, 2,4-, 2,5-dichloro-p-phenylenediamine, 2-chloro-p-phenylenediamine, N,N-bis-(2-hydroxy-ethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroxyethylamino)-4-methoxy-2-methyl-, 4-amino-2-aminomethyl phenol, 4-amino-2-hydroxymethyl phenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalene-1-sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic anilines and phenols containing another aromatic radical, such as 4,4'-diaminostilbene dihydrochloride, 4,4'-diaminostilbene-2,2'-disulfonic acid sodium salt, 4,4'-diaminodiphenyl methane, -sulfide, -sulfoxide, -amine, 4,4'-diaminodiphenylamine-2-sulfonic acid, 4,4'-diaminobenzophenone, -diphenyl ether, 3,3',4,4'-tetraaminodiphenyl tetrahydrochloride, 3,3'4,4'-tetraaminobenzophenone, 1,3-bis-(2,4-diaminophenoxy)-propane tetrahydrochloride, 1,8-bis-(2,5-diaminophenoxy)-3,6-dioxaoctane tetrahydrochloride, 1,3-bis-(4-aminophenylamino)-propane, -2-propanol, 1,3-bis-[N-(4-aminophenyl)-2-hydroxyethylamino]-2-propanol, N,N-bis-[2-(4-aminophenoxy)-ethyl]-methylamine trihydrochloride;
nitrogen-containing heterocyclic compounds selected from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethyl pyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methyl pyrimidine, 2,3,4-trimethyl pyrrole, 2,4-dimethyl-3-ethyl pyrrole, 3,5-diaminopyrazole, -1,2,4-triazole, 3-amino-, 3-amino-5-hydroxypyrazole, 2-, 3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline and indole and indoline derivatives, such as 4-, 5-, 6-, 7-aminoindole, 7-hydroxyindole, 5,6-dihydroxyindole, 5,6-dihydroxyindoline and 4-hydroxyindoline and physiologically compatible salts of these compounds formed with preferably inorganic acids;
aromatic hydroxy compounds selected from the group consisting of 2-, 4-, 5-methyl resorcinol, 2,5-dimethyl resorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalene sulfonic acid, 3,6-dihydroxy-2,7-naphthalene sulfonic acid and
CH-active compounds selected from the group consisting of 1,2,3,3-tetramethyl-3H-indolium iodide, 1,2,3,5-tetraamethyl indolium-p-toluene sulfonate, 1,2,3,5-tetramethyl-3H-indolium methane sulfonate, 2,3-dimethylbenzothiazolium iodide, 2,3-dimethylbenzothiazolium-p-toluene sulfonate, rhodanine, rhodanine-3-acetic acid, 1-ethyl(methyl)-2-quinaldinium iodide, barbituric acid, thiobarbituric acid, 1,3-dimethyl(ethyl) thiobarbituric acid, oxindole, coumaranone and 1-methyl-3-phenyl-2-pyrazolinone.

10. A preparation as claimed in claim 9, **characterized in that** component B is selected from the group consisting of N-(2-hydroxyethyl)-N-ethyl-, 2-chloro-p-phenylenediamine, N,N-bis-(2-hydroxyethyl)-p-phenylenediamine, 4-aminophenol, p-phenylenediamine, 2-(2,5-diaminophenyl)-ethanol, 2,5-diaminotoluene, 3,4-methylenedioxyaniline, 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethyl pyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5,6-dihydroxyindole and 5,6-dihydroxyindoline and physiologically compatible salts of these compounds formed with preferably inorganic acids.

11. A preparation as claimed in any of claims 8 to 10, **characterized in that** substantive dyes from the group of nitrophenylenediamines, nitroaminophenols, anthraquinones or indophenols are used.

12. A preparation as claimed in any of claims 8 to 10, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkaline earth metals, such as potassium, sodium or lithium, alkaline earth metals, such as magnesium, calcium, strontium or barium, or of aluminium, manganese, iron, cobalt, copper or zinc, are added.

13. A process for coloring keratin-containing fibers in which a colorant containing
A at least one isatin derivative corresponding to general formula I: 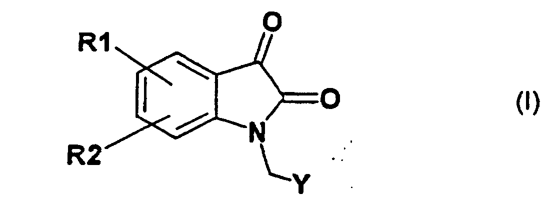 in which R¹ and R² independently of one another represent a hydrogen atom, a halogen atom, a hydroxy group, a (C₁₋₄)-alkyl, hydroxy-(C₁₋₄)-alkyl, tert.amino-(C₁₋₄)-alkyl, (C₁₋₄)-alkoxy group, an amino group optionally substituted by one or two (C₁₋₄)-alkyl or hydroxy-(C₁₋₄)-alkyl groups, a nitro, carboxy or sulfo group and
Y is a hydroxy group, a (C₁₋₄)-alkoxy group or an amino group which may be substituted by (C₁₋₄)-alkyl, (C₁₋₄)-alkoxy-(C₁₋₄)-alkyl, carboxy-(C₁₋₄)-alkyl, sulfo-(C₁₋₄)-alkyl or hydroxy-(C₁₋₄)-alkyl groups or which may be part of a heterocyclic 5-, 6- or 7-membered ring,
or physiologically compatible salts thereof and
B at least one compound containing a primary or secondary amino group or hydroxy group selected from primary or secondary aliphatic or aromatic amines, nitrogen-containing heterocyclic compounds, amino acids, oligopeptides made up of 2 to 9 amino acids and aromatic hydroxy compounds and/or at least one CH-active compound
and typical cosmetic ingredients is applied to the keratin-containing fibers, left thereon for a time, usually about 30 minutes, and then rinsed out again or washed out with a shampoo.

## Revendications

1. Utilisation de dérivés d'isatine de formule I 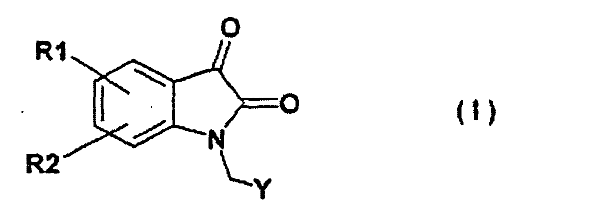 dans laquelle R¹ et R² représentent indépendamment l'un de l'autre, un atome d'hydrogène, un atome d'halogène, un groupe hydroxy, un groupe alkyle en C₁ à C₄, hydroxy(alkyle en C₁ à C₄), tert-amino(alkyle en C₁ à C₄), alcoxy en C₁ à C₄, un groupe amino, qui peut être substitué par un ou deux groupes alkyle en C₁ à C₄ ou hydroxy(alkyle en C₁ à C₄), un groupe nitro, carboxy ou un groupe sulfo, et
Y représente un groupe hydroxy, un groupe alcoxy en C₁ à C₄, ou un groupe amino, qui peut être substitué par des groupes alkyle en C₁ à C₄, (alcoxy en C₁ à C₄)-(alkyle en C₁ à C₄), carboxy(alkyle en C₁ à C₄), sulfo(alkyle en C₁ à C₄) ou hydroxy(alkyle en C₁ à C₄), ou être un constituant d'un cycle hétérocyclique de 5, 6 ou 7 maillons,
ou de sels acceptables sur le plan physiologique de ceux-ci, pour teindre des fibres kératiniques, en particulier les cheveux humains.

2. Utilisation selon la revendication 1, **caractérisée en ce que** les dérivés d'isatine de formule I sont contenus en une quantité à chaque fois de 0,03 à 65, en particulier de 1 à 40 mmoles, à chaque fois par rapport à 100 g de la teinture totale.

3. Utilisation selon la revendication 1 ou 2, **caractérisée en ce que** le dérivé d'isatine de formule I est choisi parmi la 1-hydroxyméthylisatine, la 1-hydroxyméthyl-5-méthylisatine, la 1-hydroxyméthyl-5-chloroisatine, la 1-hydroxyméthyl-5-sulfoisatine, la 1-hydroxyméthyl-5-carboxyisatine, la 1-hydroxyméthyl-5-nitroisatine, la 1-hydroxyméthyl-5-bromoisatine, la 1-hydroxyméthyl-5-méthoxyisatine, la 1-hydroxyméthyl-5,7-dichloroisatine, la 1-diméthylaminométhylisatine, la 1-diéthylaminométhyisatine, la 1-(bis-(2-hydroxyéthyl)-aminométhyl)-isatine, la 1-(2-hydroxyéthylaminométhyl)-isatine, la 1-(bis-(2-hydroxypropyl)-aminométhyl)-isatine, la 1-pyrrolidinométhylisatine, la 1-pipéridinométhylisatine, la 1-morpholinométhylisatine, la 1-(1,2,4-triazolyl)-méthylisatine, la 1-(1-imidazolyl)-méthylisatine, la 1-carboxyméthyl-aminométhylisatine, la 1-(2-carboxyéthylaminométhyl)-isatine, la 1-(3-carboxy-propylaminométhyl)-isatine, la 1-(bis-(2-hydroxyéthyl)-aminométhyl)-5-méthylisatine, la 1-pipéridinométhyl-5-chloroisatine, la 1-(2-sulfoéthylamino)-isatine, ainsi que les sels de métal alcalin et d'ammonium avec les composés acides.

4. Utilisation selon l'une quelconque des revendications 1 à 3, **caractérisée en ce qu'**en sus, au moins un composé ayant un groupe amino primaire ou secondaire ou un groupe hydroxy, choisi parmi les amines aliphatiques ou aromatiques, primaires ou secondaires, les composés hétérocycliques azotés, les acides aminés, les oligopeptides construits à partir de 2 à 9 acides aminés, et les composés hydroxyaromatiques, et/ou au moins un composé à groupe CH actif sont mis en oeuvre.

5. Utilisation selon l'une quelconque des revendications 1 à 4, **caractérisée en ce que** des agents d'oxydation sont mis en oeuvre en une quantité de 0,01 à 6% en poids, par rapport à la solution d'application.

6. Utilisation selon la revendication 5, **caractérisée en ce que** H₂O₂ est mis en oeuvre en tant qu'agent d'oxydation.

7. Utilisation selon l'une quelconque des revendications 1 à 6, **caractérisée en ce que** des tensio-actifs anioniques, zwitterioniques et/ou non ioniques, sont mis en oeuvre.

8. Produit pour teindre des fibres kératiniques, en particulier les cheveux humains, contenant
A au moins un dérivé d'isatine de formule I, 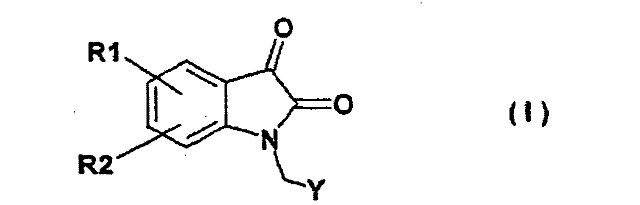 dans laquelle R¹ et R² représentent indépendamment l'un de l'autre, un atome d'hydrogène, un atome d'halogène, un groupe hydroxy, un groupe alkyle en C₁ à C₄, hydroxy(alkyle en C₁ à C₄), tert-amino(alkyle en C₁ à C₄), alcoxy en C₁ à C₄, un groupe amino, qui peut être substitué par un ou deux groupes alkyle en C₁ à C₄ ou hydroxy-(alkyle en C₁ à C₄), un groupe nitro, carboxy ou un groupe sulfo, et
Y représente un groupe hydroxy, un groupe alcoxy en C₁ à C₄, ou un groupe amino, qui peut être substitué par des groupes alkyle en C₁ à C₄, (alcoxy en C₁ à C₄)-(alkyle en C₁ à C₄), carboxy-(alkyle en C₁ à C₄), sulfo-(alkyle en C₁ à C₄) ou hydroxy-(alkyle en C₁ à C₄), ou être un constituant d'un cycle hétérocyclique de 5, 6 ou 7 maillons,
ou de sels acceptables sur le plan physiologique de ceux-ci et
B au moins un composé avec un groupe amino primaire ou secondaire ou un groupe hydroxy, choisi parmi les amines aliphatiques ou aromatiques, primaires ou secondaires, les composés hétérocycliques azotés, les acides aminés, les oligopeptides construits à partir de 2 à 9 acides aminés et les composés hydroxyaromatiques, et/ou au moins un composé à groupe CH actif.

9. Produit selon la revendication 8, **caractérisé en ce que** le composant B est choisi parmi
les amines primaires ou secondaires issues du groupe formé par la N-(2-hydroxyéthyl)-N-éthyl-, N-(2-méthoxyéthyl-), 2,3-, 2,4-, 2,5-dichloro-p-phénylènediamine, la 2-chloro-p-phénylènediamine, la N,N-bis-(2-hydroxyéthyl)-p-phénylènediamine, le dibromhydrate de 2,5-dihydroxy-4-morpholinoaniline, le 2-, 3-, 4-aminophénol, la o-, m-, p-phénylènediamine, le 2,4-diaminophénoxyéthanol, le 2-(2,5-diaminophényl)-éthanol, le 2,5-diaminotoluène, -phénol, -phénéthol, la 4-méthylamino-, 3-amino-4-(2'-hydroxyéthyloxy)-, 3,4-méthylènediamino-, 3,4-méthylènedioxyaniline, le 3-amino-2,4-dichloro-, 4-méthylamino-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 5-(2-hydroxyéthylamino)-4-méthoxy-2-méthyl-, 4-amino-2-aminométhyl-phénol, le 4-amino-2-hydroxyméthylphénol, le 1,3-diamino-2,4-diméthoxybenzène, l'acide 2-, 3-, 4-aminobenzoïque, -aminophénylacétique, l'acide 2,3-, 2,4-, 2,5-, 3,4-, 3,5-diaminobenzoïque, l'acide 4-, 5-aminosalicylique, l'acide 3-amino-4-hydroxy-, 4-amino-3-hydroxy-benzoïque, l'acide 2-, 3-, 4-aminobenzènesulfonique, l'acide 3-amino-4-hydroxybenzènesulfonique, l'acide 4-amino-3-hydroxynaphtalène-sulfonique, l'acide 6-amino-7-hydroxynaphtalène-2-sulfonique, l'acide 7-amino-4-hydroxynaphatalène-2-sulfonique, l'acide 4-amino-5-hydroxynaphtalène-2,7-disulfonique, l'acide 3-amino-2-napthoïque, l'acide 3-aminophtalique, l'acide 5-aminoisophtalique, le 1,3,5-, 1,2,4-triaminobenzène, le tétrachlorhydrate de 1,2,4,5-tétraaminobenzène, le trichlorhydrate de 2,4,5-triaminophénol, le pentachlorhydrate de pentaaminobenzène, l'hexachlorhydrate d'hexaaminobenzène, le trichlorhydrate de 2,4,6-triaminorésorcine, le sulfate de 4,5-diaminopyrocatéchol, le dichlorhydrate de 4,6-diaminopyrogallol, le sulfate de 3,5-diamino-4-hydroxypyrocatéchol, les anilines aromatiques ou les phénols, avec un résidu aromatique supplémentaire comme le dichlorhydrate de 4,4'-diaminostilbène, l'acide 4,4'-diaminostilbène-2,2'-disulfonique, le sel sodique, le 4,4'-diaminodiphénylméthane, le sulfure de 4,4'-diaminodiphényle, le sulfoxyde de 4,4'-diaminodiphényle, la 4,4'-diaminodiphénylamine, l'acide 4,4'-diaminodiphénylamine-2-sulfonique, la 4,4'-diaminobenzophénone, -diphényléther, le tétrachlorhydrate de 3,3', 4,4'-tétraaminodiphényle, la 3,3', 4,4'-tétraamino-benzophénone, le tétrachlorhydrate de 1,3-bis-(2,4-diaminophénoxy)-propane, le tétrachlorhydrate de 1,8-bis-(2,5-diamino-phénoxy)-3,6-dioxa-octane, le 1,3-bis-(4-aminophénylamino)-propane, le 1,3-bis-(4-aminophénylamino)-2-propanol, le 1,3-bis-[N-(4-aminophényl)-2-hydroxyéthylamino]-2-propanol, le trichlorhydrate de N,N-bis-[2-(4-amino-phénoxy)-éthyl]méthylamine,
les composés hétérocycliques azotés choisis dans le groupe formé par la 2-, 3-, 4-amino-, 2-amino-3-hydroxy-, 2,6-diamino-, 2,5-diamino-, 2,3-diamino-, 2-diméthylamino-5-amino-, 3-amino-2-méthylamino-6-méthoxy-, 2,3-diamino-6-méthoxy-, 3,5-diamino-2,6-diméthoxy-, 2,4,5-triamino-, 2,6-dihydroxy-3,4-diméthylpyridine, la 4,5,6-triamino-, 2-hydroxy-4,5,6-triamino-, 4-hydroxy-2,5,6-triamino-, 2,4,5,6-tétraamino-, 2-méthylamino-4,5,6-triamino-, 2,4-, 4,5-diamino-, 2-amino-4-méthoxyméthylpyrimidine, le 2,3,4-triméthyl-pyrrole, le 2,4-diméthyl-3-éthyl-pyrrole, le 3,5-diaminopyrazole, le 3,5-diamino-1,2,4-triazole, le 3-amino-, 3-amino-5-hydroxypyrazole, la 2-, 3-, 8-amino-quinoléine, la 4-aminoquinaldine, l'acide 2-, 6-aminonicotinique, la 5-aminoisoquinoléine, le 5-, 6-aminoindazole, le 5-, 7-amino-benzimidazole, le 5-, 7-amino-benzothiazole, la 2,5-dihydroxy-4-morpholinoaniline, ainsi que les dérivés d'indole et d'indoline, comme le 4-, 5-, 6-, 7-aminoindole, le 4-, 5-, 6-, 7-hydroxyindole, le 5,6-dihydroxyindole, la 5,6-dihydroxyindoline et la 4-hydroxyindoline, ainsi qu'à chaque fois les sels de ces composés, acceptables sur le plan physiologique, formés de préférence avec des acides inorganiques,
les composés hydroxyaromatiques, choisis dans le groupe formé par la 2-, 4-, 5-méthylrésorcine, la 2,5-diméthylrésorcine, la résorcine, le 3-méthoxyphénol, le pyrocatéchol, l'hydroquinone, le pyrogallol, la phloroglucine, l'hydroxyhydroquinone, le 2-, 3-, 4-méthoxy-, 3-diméthylamino-, 2-(2-hydroxy-éthyl)-, 3,4-méthylènedioxyphénol, l'acide 2,4-, 3,4-dihydroxybenzoïque, l'acide 2,4-, 3,4-dihydroxyphénylacétique, l'acide gallique, l'acide 2,4,6-trihydroxybenzoïque, la 2,4,6-trihydroxyacétophénone, la 2-,4-chlororésorcine, le 1-naphtol, le 1,5-, 2,3-, 2,7-dihydroxynapthalène, l'acide 6-diméthylamino-4-hydroxy-2-napthalène-sulfonique, l'acide 3,6-dihydroxy-2,7-naphtalène-sulfonique, et les composés à groupe CH actif choisis dans le groupe formé par l'iodure de 1,2,3,3-tétraméthyl-3H-indolinium, le p-toluènesulfonate de 1,2,3,5-tétraméthylindolinium, le méthanesulfonate de 1,2,3,5-tétraméthylindolinium, l'iodure de 2,3-diméthyl-benzothiazolium, le p-tolènesulfonate de 2,3-diméthyl-benzothiazolium, la rhodanine, l'acide rhodanine-3-acétique, l'iodure de 1-éthyl-(méthyl)-2-quinaldinium, l'acide barbiturique, l'acide thiobarbiturique, l'acide 1,3-diméthyl(éthyl)thiobarbiturique, l'oxindole, la coumaranone, et la 1-méthyl-3-phényl-2-pyrazolinone.

10. Produit selon la revendication 9, **caractérisé en ce que** le composant B est choisi dans le groupe formé par la N-(2-hydroxyéthyl)-N-éthyl-, 2-chloro-p-phénylènediamine, la N,N-bis-(2-hydroxyéthyl)-p-phénylènediamine, le 4-aminophénol, la p-phénylènediamine, le 2-(2,5-diaminophényl)-éthanol, le 2,5-diaminotoluène, la 3,4-méthylènedioxyaniline, le 3-amino-2,4-dichloro-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 2-aminométhyl-4-aminophénol, le 2-hydroxyméthyl-4-aminophénol, le 3,4-méthylènedioxyphénol, l'acide 3,4-diaminobenzoïque, la 2,5-diamino-, 2-diméthylamino-5-amino-, 3-amino-2-méthylamino-6-méthoxy-, 2,3-diamino-6-méthoxy, 3,5-diamino-2,6-diméthoxy-, 2,6-dihydroxy-3,4-diméthylpyridine, la 2-hydroxy-4,5,6-triamino-, 4-hydroxy-2,5,6-triamino-, 2,4,5,6-tétraamino-, 2-méthylamino-4,5,6-triamino-pyrimidine, le 3,5-diaminopyrazole, le 3-amino-5-hydroxy-pyrazole, le 5,6-dihydroxyindole et la 5,6-dihydroxyindoline, ainsi qu'à chaque fois les sels de ces composés, acceptables sur le plan physiologique, formés de préférence avec des acides inorganiques.

11. Produit selon l'une quelconque des revendications 8 à 10, **caractérisé en ce que** des colorants montant directement sur la fibre, issus du groupe des nitrophénylènediamines, des nitroaminophénols, des anthraquinones, ou des indophénols sont mis en oeuvre.

12. Produit selon l'une quelconque des revendications 8 à 11, **caractérisé en ce que** des sels d'ammonium ou métalliques y sont ajoutés, choisis dans le groupe des formiates, carbonates, halogénures, sulfates, butyrates, valériates, caproates, acétates, lactates, glycolates, tartrates, citrates, gluconates, propionates, phosphates et phosphonates de métaux alcalins, comme le potassium, le sodium ou le lithium, de métaux alcalino-terreux comme le magnésium, le calcium, le strontium, ou le baryum, ou d'aluminium, de manganèse, de fer, de cobalt, de cuivre ou de zinc.

13. Procédé pour teindre des fibres kératiniques, dans lequel une teinture contenant
A au moins un dérivé d'isatine de formule I, 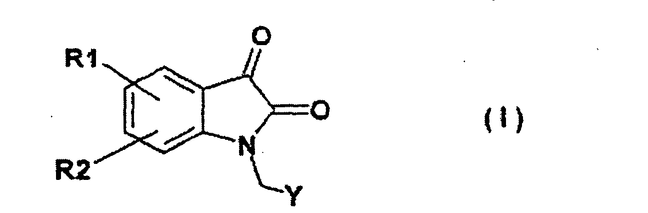 dans laquelle R¹ et R² représentent indépendamment l'un de l'autre, un atome d'hydrogène, un atome d'halogène, un groupe hydroxy, un groupe alkyle en C₁ à C₄, hydroxy(alkyle en C₁ à C₄), tert-amino(alkyle en C₁ à C₄), alcoxy en C₁ à C₄, un groupe amino, qui peut être substitué par un ou deux groupes alkyle en C₁ à C₄ ou hydroxy-(alkyle en C₁ à C₄), un groupe nitro, carboxy ou un groupe sulfo, et
Y représente un groupe hydroxy, un groupe alcoxy en C₁ à C₄, ou un groupe amino, qui peut être substitué par des groupes alkyle en C₁ à C₄, (alcoxy en C₁ à C₄)-(alkyle en C₁ à C₄), carboxy-(alkyle en C₁ à C₄), sulfo-(alkyle en C₁ à C₄) ou hydroxy-(alkyle en C₁ à C₄), ou être un constituant d'un cycle hétérocyclique de 5, 6 ou 7 maillons,
ou des sels acceptables sur le plan physiologique de ceux-ci et
B au moins un composé avec un groupe amino primaire ou secondaire ou un groupe hydroxy, choisi parmi les amines aliphatiques ou aromatiques, primaires ou secondaires, les composés hétérocycliques azotés, les acides aminés, les oligopeptides construits à partir de 2 à 9 acides aminés, les composés hydroxyaromatiques, et/ou au moins un composé à groupe CH actif,
ainsi que des ingrédients cosmétiques habituels, est appliquée sur les fibres kératiniques, est laissée quelque temps, habituellement environ 30 minutes sur les fibres, puis elle est éliminée par rinçage ou par lavage avec un shampoing.
